(19) Europäisches Patentamt
European Patent Office
Office européen des brevets

(11) Publication number: **0 066 130**
**A1**

(12) **EUROPEAN PATENT APPLICATION**

(21) Application number: 82104091.2

(22) Date of filing: 12.05.82

(51) Int. Cl.³: C 07 C 119/048
C 07 C 35/04
//C08G18/80

(30) Priority: 22.05.81 US 266555

(43) Date of publication of application: 08.12.82 Bulletin 82/49

(84) Designated Contracting States: BE DE FR GB IT

(71) Applicant: Mobay Chemical Corporation
Penn Lincoln Parkway West
Pittsburgh, Pennsylvania 15205(US)

(72) Inventor: Slack, William E.
R.D. 1 Box 100
Moundsville West Virginia 26041(US)

(74) Representative: Weber, Gerhard, Dr. et al,
BAYER AG Zentralbereich Patente, Marken und Lizenzen
D-5090 Leverkusen, Bayerwerk(DE)

(54) **2,2,4,4-Tetraalkyl-1,3-cyclobutanediol modified diphenylmethane diisocyanate.**

(57) The instant invention is directed to a process for the production of diisocyanate compounds which are both stable and liquid at room temperature, comprising reacting a diisocyanate selected from the group consisting of 2,2'-diphenylmethane diisocyanate, 2,4'-diphenylmethane diisocyanate, 4,4'-diphenylmethane diisocyanate and mixtures thereof with 2,2,4,4-tetraalkylcyclobutanediol at a temperature of from about 40°C to about 120°C in such a ratio as to afford a product having a percent NCO value in the range of 20 to 30%. The instant invention is also directed to the product prepared by this process.

EP 0 066 130 A1

# 2,2,4,4-TETRAALKYL-1,3-CYCLOBUTANEDIOL MODIFIED DIPHENYLMETHANE DIISOCYANATE

## BACKGROUND OF THE INVENTION

This invention relates to organic diisocyanates and mixtures of organic diisocyanates based on 2,2'-, 2,4'- and/or 4,4'-diphenylmethane diisocyanates which are liquid at room temperature. The invention also relates to a process for preparing these diisocyanates.

Diisocyanates which are liquid at room temperature have numerous advantages over solid diisocyanates because they are easier to mix and work with. Diisocyanates, however, which are liquid at room temperature, are as a rule physiologically harmful because of their high vapor pressure and can only be handled if certain precautions are taken. For this reason, various attempts have been made to start with diisocyanates that are solid at room temperature and convert these into the liquid form by certain measures. However, one usually obtains isocyanates with high functionality, i.e., tri- or polyisocyanates or higher molecular weight diisocyanates or a combination of these.

The most important diisocyanates which are solid at room temperature and which are readily available on a large commercial scale are 4,4'-diphenylmethane diisocyanate and the 2,4'-isomer thereof which melt at 39°C and 34.5°C, respectively. Attempts have been made also to liquefy the 4,4'-diphenylmethane diisocyanate isomer. Thus, for example, according to U.S. Patent 3,152,162, 4,4'-diphenylmethane diisocyanate is heated at temperatures above 150°C to affect a partial carbodiimization of the isocyanate. The isocyanate groups still present partly react with the resulting carbodiimide groups to form uretone imine. The resultant liquid products are not diisocyanates but are polyisocyanates. This manifests itself in the reaction of the products with bifunctional low molecular weight or higher molecular weight hydroxyl compounds, the reaction

leading not to soluble linear products but to insoluble cross-linked products.

It is known from U.S. Patent 3,644,457 to react 4,4'- and/or 2,4'-diphenylmethane diisocyanate with a branched aliphatic dihydroxy compound which yields a product which is liquid at room temperature.

It is therefore an object of this invention to provide improved liquid organic diisocyanates which are liquid at room temperature. Another object of this invention is to provide a mixture of diphenylmethane diisocyanates which is liquid at room temperature. A further object of this invention is to provide organic diisocyanates which remain liquid even on prolonged storage at low temperatures. Still another object of this invention is to provide an improved process for preparing liquid organic diisocyanates.

DESCRIPTION OF THE INVENTION

The instant invention is directed to a process for preparing a liquid modified diphenylmethane diisocyanate comprising reacting a 2,2,4,4-tetraalkylcyclobutanediol and preferably, 2,2,4,4-tetramethylcyclobutanediol, with diphenylmethane diisocyanate at a temperature between about 40 to 120°C, preferably between 40 to 100°C, more preferably between 45 to 80°C, and most preferably between 50 to 60°C in such a ratio as to produce a product having a weight percent NCO value in the range of 20 to 30%, and preferably in the range of 21 to 27%. In general, the weight ratio of diphenylmethane diisocyanate to diol will be from 15:1 to 5:1 and is preferably from 10:1 to 6:1. The liquid modified diphenylmethane diisocyanate may also be prepared as a concentrate (that is, with a lower percent NCO) and diluted with additional diphenylmethane diisocyanate to adjust the percent NCO to the desired value.

The instant invention is also directed to diisocyanate compounds which are both stable and liquid at room temperature, prepared by reacting a diphenylmethane diisocyanate with a 2,2,4,4-tetraalkylcyclobutanediol at a temperature of from about 40°C to about 120°C in such a ratio as to afford

a product having a weight percent NCO value in the range of 20 to 30%. As used herein, the term "diphenylmethane diisocyanate" is defined as 2,2'-diphenylmethane diisocyanate, 2,4'-diphenylmethane diisocyanate, 4,4'-diphenylmethane diisocyanate and mixtures thereof.

Most short chain diols, such as 1,4- and 1,3-butanediol, ethylene glycol, and the like when reacted with diphenylmethane diisocyanate result in solids at room temperature. In contrast, the instant liquid modified diphenylmethane diisocyanate remains a liquid at 25°C. The process of the instant invention may also be used to increase the hard block concentration via the prepolymer of the RIM formulation instead of increasing the butanediol concentration in the polyol side which leads to mixing problems. Heat sag values are specially improved when the instant prepolymers are used.

The liquid diisocyanates which can be prepared according to the invention, preferably from 4,4'- and/or 2,4'-diphenylmethane diisocyanate, have a very low viscosity and can therefore be processed very easily. For example, they can be cast or metered through pumps. Additionally, they have a very low vapor pressure and are therefore less physiologically harmful.

The process may be carried out by introducing the 2,2,4,4-tetraalkylcyclobutanediol into the diisocyanate at temperatures of about 40 to about 120°C with stirring. The isocyanate content of the products of the process amounts to about 20% by weight NCO to about 30% by weight NCO, and preferably 21 to 27%.

2,2,4,4-tetraalkylcyclobutanediols wherein each alkyl group, which may be the same or different, contains from 1 to 7 carbon atoms are preferably used. The presently preferred compound is 2,2,4,4-tetramethylcyclobutanediol.

It is also possible to blend the tetraalkylcyclobutanediol of the present invention with other hydroxy functional materials. If the hydroxyl functional material

to be blended is of the type which forms liquid products with diphenylmethane diisocyanate (such as those described in U.S. Patents 3,644,457; 4,115,429 and 4,118,411), as little as 10% by weight (based on the weight of the tetraalkylcyclobutanediol and other hydroxy functional materials) of the tetraalkylcyclobutanediol can be used. Where the hydroxy functional material does not normally form a liquid product with diphenylmethane diisocyanate, the tetraalkylcyclobutanediol should constitute at least 50% by weight of the mixture of the tetraalkylcyclobutanediol with other hydroxy functional materials. It is generally preferred, however, to use the tetraalkylcyclobutane alone.

The products of the process can be used for all sorts of different polyaddition reactions in the lacquer and plastics industry. For example, they may be used in the production of polyurethane foams or polyurethane elastomers which are in turn useful for the preparation of cushions or cast gear wheels, respectively. The invention is further illustrated by the following Example.

## EXAMPLE

### EXAMPLE 1

About 850 g of 4,4'-diphenylmethane diisocyanate (97% 4,4'-MDI and 3% 2,4'-MDI) are melted and mixed at about 58 to 73°C with about 85.9 g of 2,2,4,4-tetramethylcyclobutanediol with stirring for about 3 hours. The resulting product had an NCO value of 25%. The viscosity was 367 cPs at 25°C.

Although the invention has been described in considerable detail in the foregoing, it is to be understood that such detail is solely for the purpose of illustration and that many variations can be made by those skilled in the art without departing from the spirit and scope of the invention except as set forth in the claims.

WHAT IS CLAIMED IS:

1. A process for the production of diisocyanate compounds which are both stable and liquid at room temperature, comprising reacting a diphenylmethane diisocyanate with a 2,2,4,4-tetraalkylcyclobutanediol at a temperature of from about 40°C to about 120°C in such a ratio as to yield a product having a weight percent NCO value of from 20 to 30%.

2. The process of Claim 1, wherein said 2,2,4,4-tetraalkylcyclobutanediol is of the formula:

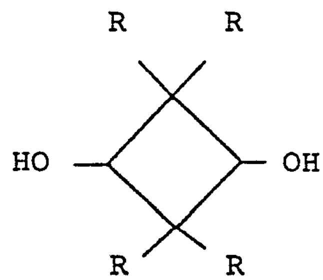

wherein R, which may be the same or different, contains from 1 to 7 carbon atoms.

3. The process of Claim 2, wherein said 2,2,4,4-tetraalkylcyclobutanediol is 2,2,4,4-tetramethylcyclobutanediol.

4. The process of Claim 2, wherein the percent NCO value of the product is from 21 to 27%.

5. The process of Claim 1, wherein a concentrate is formed and subsequently diluted with diisocyanate to the claimed NCO value.

6. The process of Claim 1, wherein the reaction temperature is 40 to 100°C.

7. The process of Claim 6, wherein said reaction temperature is 45 to 80°C.

8. The process of Claim 7, wherein said reaction temperature is 50 to 60°C.

9. Diisocyanate compounds having an NCO content of from 20 to 30%, which are both stable and liquid at room temperature, prepared by reacting a diphenylmethane diisocyanate with a 2,2,4,4-tetraalkylcyclobutanediol at a temperature of from about 40°C to about 120°C.

0066130

European Patent Office

# EUROPEAN SEARCH REPORT

Application number

EP 82 10 4091.2

## DOCUMENTS CONSIDERED TO BE RELEVANT

| Category | Citation of document with indication, where appropriate, of relevant passages | Relevant to claim |
|---|---|---|
| Y | Chemical Abstracts vol. 68, no.6, 5 February,1968 Columbus, Ohio, USA S. YAMASHIRO et al. "Polyurethane elastomers " page 2207, column 2, abstract no. 22683f & JP - 67 - 19279 -- | 1-9 |
| D,Y | US - A - 4 118 411 (MOBAY CHEMICAL CORP.) * claims * -- | 1-9 |
| D,Y | US - A - 3 644 457 (BAYER AG) * claims * -- | 1-9 |
| Y | DE - A - 2 258 211 (I.C.I.) * formula (c) ; Examples 5, 6, 15 * & GB - A - 1 389 700 -- | 1-9 |
| Y | Chemical Abstracts vol. 64, no. 7 28 March, 1966 Columbus, Ohio, USA UNION CARBIDE CORP. "Plyurethans" column 9917 & GB - A - 1 012 929 ---- | 1-9 |

**CLASSIFICATION OF THE APPLICATION (Int. Cl. 3)**

C 07 C 119/048
C 07 C 35/04
//C 08 G 18/80

**TECHNICAL FIELDS SEARCHED (Int.Cl. 3)**

C 07 C 35/04
C 07 C 119/00
C 08 G 18/00

**CATEGORY OF CITED DOCUMENTS**

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

X The present search report has been drawn up for all claims

| Place of search | Date of completion of the search | Examiner |
|---|---|---|
| Berlin | 21-07-1982 | BREW |

EPO Form 1503.1 06.78